(19) Europäisches Patentamt
European Patent Office
Office européen des brevets

(11) Publication number: 0 275 340 B1

(12) # EUROPEAN PATENT SPECIFICATION

(45) Date of publication of the patent specification: 22.08.90

(51) Int. Cl.⁵: A61K 31/19

(21) Application number: 87100791.0

(22) Date of filing: 21.01.87

(54) **An aqueous acidic contraceptive composition and a method for its preparation.**

(43) Date of publication of application: 27.07.88 Bulletin 88/30

(45) Publication of the grant of the patent: 22.08.90 Bulletin 90/34

(84) Designated Contracting States: AT CH DE FR GB IT LI SE

(56) References cited:
GB-A- 2 112 285
US-A- 4 665 096

(73) Proprietor: Oraa de Cova, Maritza, Avenida Nivaldo Res. Mochima, 1er Piso, LB, Urbanizacion Avila La Florida Caracas(VE)

(72) Inventor: Oraa, Espartaco, Deceased(VE)

(74) Representative: Patentanwälte Dipl.-Ing. Conrad Köchling Dipl.-Ing. Conrad-Joachim Köchling, Fleyer Strasse 135, D-5800 Hagen 1(DE)

EP 0 275 340 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

ACTORUM AG

## Description

### Field of the Invention

My present invention relates to a contraceptive, composition which, when applied intravaginaly immediately after coitus, is significantly more effective than many of the contraceptive methods widely in use today and which can, because of the comparative low cost of its ingredients, be made available pratically anywhere in the world at a relatively low cost, but nevertheless with high efficiency.

### Background of the Invention

There are a wide variety of contraceptive agents on the market and in general use, some of which require post-coital introduction into the vagina and can be considered spermaticides, working by effectively destroying the sperm in the vagina before they pass the cervix and create the possibility of fertilization of an ovum.

Various jellies or other compositions have been used for this purpose and other contraceptive methods such as the intrauterine device, which frequently causes irritation and discharge from the uterus, the diaphragm which is often uncomfortable, and the oral contraceptive which may be hormonally based and hence undesirable, have all been used with varying degrees of sucess, with greater or lesser amounts of discomfort, and at greater or lesser cost per application.

Further there is known a composition for intimate feminine hygiene, which uses glacial acetic acid in a vaginal douche composition, as disclosed by GB-A 2 112 285.

In spite of the fact that family planning is becoming increasingly more important as the world population grows and the drain on natural resources increases, and in spite of the fact that considerable research effort has been expended on various contraceptive techniques and operations, there has been, to my knowledge, no truly effective low-cost post-coitally applicable contraceptive method or composition which is substantially free of discomfort and yet virtually assures total destruction of the sperm following coitus.

### Objects of the Invention

It is, therefore, the principal object of the present invention to provide an improved post-coital method of contraception which utilizes a comparatively low-cost composition consisting entirely of constituents which are safe for any user, and which nevertheless is substantially one hundred percent effective in preventing fertilization of an ovum.

Another object of this invention is to provide an improved contraceptive composition which obviates the disadvantages of earlier contraceptive compositions and which have been used without the pronounced disadvantages of mechanical contraceptive techniques.

Yet another object of my invention is to provide a device for administering the composition in the improved contraceptive method of the present invention.

### Summary of the Invention

These objects and others, which will become apparent hereinafter, are attained in accordance with the present invention which comprises an aqueous acidic contraceptive composition characterized in that it consits essentially of an aqueous solution containing 1.2 ml ± 10% of 2-hydroxy-propanoic acid 0.9 ml ± 10% of the 3-hydroxy-3-carboxy-pentanodioic acid and 0.5 ml ± 10% ofglacial acetic acid per 100 ml, said solution being adjested to a pH of 3.2 to 3.5 and a method of making such an aqueours acidic contraceptive composition contraceptive composition characterized in that it comprises the steps of dissolving 1.2 ml ± 10% of 2-hydroxy-propanoic acid, 0.9 ± 10% of 3-hydroxy-3-carboxy-pentanodioic acid and 0.5 ml ± 10% of glacial acetic in water to bring the solution to 100 ml and adjusting the pH thereof to3.2 to 3.5 with a sodium hydroxide solution.

Following coitus and preferably immediately after coitus, i.e. no more than 5 minutes after coitus, the vagina is irrigated with such an aqueous solution of an effective amount 2-hydroxy-propanoic acid

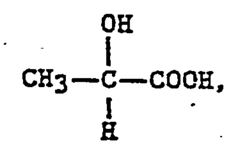

an effective amount of 3-hydroxy-3-carboxy-pentanodioic

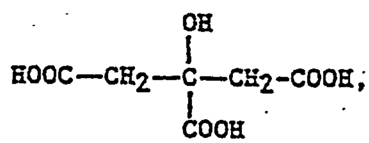

and an effective amount of acetic $CH_3COOH$ in a synergistic combiantion and preferably with the contentration of 2-hydroxy-propanoic acid being about 1.2 ml per 100 ml of the solution, the concentration of the 3-hydroxy-3-carboxy-pentanedioic acid being 0.9 ml per 100 ml of the solution and the acetic acid concentration being equivalent to 0.5 ml of glacial acetic acid per 100 ml of the solution. Water makes up the balance of the solution and for a scent, about 4 ml of the water may be constituted by rose water.

The solution is adjusted to a pH of 3.2 to 3.5 with 10 N sodium hydroxide (NaOH). The proportions of the active ingredients, namely the 2-hydroxy-propanoic acid, the 3-hydroxy-3-carboxy-pentandedioic acid and the acetic acid preferably are precisely as given above although deviations of ± 10% can be used.

The composition can be fabricated simply by mixing the components together and adding the sodium

hydroxide to bring about the pH up to the level indicated.

Surprisingly, while each or some of the active ingredients have been used as spermaticides in the past, for the concentrations given, none is totally effective and indeed no combination of two of them is totally effective, thereby indicating that all three components, when taken together, manifest a synergistic effect.

Interestingly enough, and equally surprising, is the fact that the 2-hydroxy-propanoic acid is a naturally occuring substance present in the vagina, having a protective effect against vaginal infections and is formed in the vagina apparently by fermentation of the glucose by the Doderlein bacillus.

The 3-hydroxy-3-carboxy-pentanodioic acid, likewise, is a naturally occurring bodily substance and, quite surprisingly, is found in the male genital tract and appears to have the function of liquefying semen once it has been ejaculated.

The acetic acid, of course, is a compound produced in normal metabolism. Thus all of the active ingredients are present as naturally occurring compounds in the body. That, presumably, is why the compound can be utilized without adverse effects repeatedly and with total effectiveness.

The composition is administered by a vaginal irrigator characterized by the fact that it has an elongated tube which can be inserted into the vagina and is formed remote from its discharge end with a disc which temporarily presses on the tissue surrounding the mouth of the vagina and blocks escape of the solution which is injected through this device into the vagina.

Generally speaking, 8 to 10 ml of the contraceptive solution is sufficient to fill and encompass the complete vaginal space, although slightly more of the solution, up to, say, 12 ml may be used if the vagina has been distended owing to childbirth.

The disc is preferably oval to fit effectively against the tissue surrounding the mouth of the vagina.

A surprising characteristic of the composition, demonstrated by tests in vitro and in vivo is that the composition does not change in character when it is mixed with vaginal discharge. The composition is effective, regardless of the quantity of spermatozoons, all sperm are destroyed.

## Brief Description of the Drawing

The above and other objects, features and advantages of the present invention will become more readily apparent from the following description, reference being made to the accompanying drawing in which:

Fig. 1 is an exploded view of a vaginal irrigator for applying the contraceptive composition of the present invention;

Fig. 2 is an end view of this irrigator;

Fig. 3 is a side-elevational view of an irrigator according to another feature of the invention;

Fig. 4 is a side-elevational view of a portion of this latter irrigator;

Fig. 5 is an elevational view of the disk forming a part thereof; and

Fig. 6 is a section of another part of the device shown in Fig. 3.

## Specific Description

In Fig. 1 I have shown a vaginal irrigator which comprises a tube 10 provided with a narrow bore 11 extending through the entire length thereof axially and formed at one end with a rounded tip 12 which eases insertion of the irrigator into the vagina, the passages 11 opening at this end.

At its opposite end, the passage 11 is formed with a socket 13 of the Luer-lock type to which the projection 14 of a syringe 15 may be affixed.

The irrigator is divided into an intravaginal section to the left and an extravaginal section to the right by an oval disc 16 which has been shown in greater detail in Fig. 2.

In use, the syringe 15 can be filled from a bottle containing the solution of the present invention and may be calibrated so that the desired quantity, say 10 ml, of the solution is drawn into the syringe.

The syringe is then affixed to the irrigator and the irrigator is inserted, immediately after coitus, in the vagina so that the disk 16 presses against the labia to block escape of the irrigation solution which can then be injected by displacing the plunger 17 to the left. The duct or passage 11 can be dimensioned so that less than a ml remains therein. The solution is held in place in the vagina for the 2 to 3 minutes required for total spermaticide.

I have found that venereal disease transfer to the woman can be reduced if the solution is permitted to remain in the vagina for a period of 5 to 10 minutes.

The irridator shown in Fig. 1 can be composed of plastic and need only be used in hot water and dried to prepare it for the next use.

In Figs. 3 - 6 I have shown an irrigator which can be composed of stainless steel an in which the intravaginal section 21 and the extravaginal section 22 have been joined by a thread connection 23, 24, clamping the disk 25 between them.

This unit can be cleared by disassembling if for storing although it is used in the manner which has already been described.

## Specific Example

The aqueous acidic composition 1 prepared by mixing 1.2 ml of 2 hydroxy-propanoic acid, 0.9 ml of 3 hydroxy-3-carboxy pentanedioic acid, 0.5 ml of acetic acid (glacial), 4 ml of rose water, and distilled water to bring the solution to 100 ml. Drop by drop, 10 N sodium hydroxy de is added at a pH of 3.2 to 3.5.

Immediately after coitus, 10 ml of this solution are administered using the device in Fig. 1 and held in the vagina for a period of 2 to 3 minutes. Smears show no surviving sperm.

Tests were carried out on over a 1000 in vivo

specimens under the microscope using fresh human semen and in all cases administration of solution killed all spermatozoons.

242 couples voluntarily submitted to tests, the semen having been contributed by the male partner and the female partners having been tested previously to be certain that they were not pregnant. All tests and examinations were carried out in my laboratory and outpatient clinic.

Once it was proven without a doubt that the woman were not pregnant, during their ovulation period, the semen of the respective male partner was introduced and, in the manner described above, the contraceptive solution was applied.

25 to 35 days later all were given approved pregnancy slide tests and none of the test subjects was pregnant. There were no side effects.

The solution was used voluntarily by 28 women between 18 and 30 years of age for more than 26 consecutive days and retained in the vagina for 10 minutes on each application.

No secondary effects were observed.

The solution does not appear to lose its effectiveness or to deteriorate at ambient temperature and does not need refrigeration. It is transparent and no sedimentation is observed. The solution does not stain clothing or the skin, and is not affected by vaginal flow. I observed no allergic phenomenon or any other secondary effect and the only sensation which appears to be a concomittant of its use is a mildly freshening sensation upon application.

## Claims

1. An aqueous acidic contraceptive composition characterized in that it consists essentially of an aqueous solution containing 1.2 ml ±10% of 2-hydroxy-propanoic acid, 0.9 ml ±10% of 3-hydroxy-3-carboxy-pentanodioic acid, and 0.5 ml ±10% of glacial acetic acid per 100 ml, said solution being adjusted to a pH of 3.2 to 3.5.

2. A method of making an aqueous acidic contraceptive composition according to claim 1 characterized in that it comprises the steps of dissolving 1.2 ml ±10% of 2-hydroxy-propanoic acid, 0.9 ml ±10% of 3-hydroxy-3-carboxy-pentanodioic acid and 0.5 ml ±10% of glacial acetic acid in water to bring the solution to 100 ml and adjusting the pH thereof to 3.2 to 3.5 with a sodium hydroxide solution.

## Patentansprüche

1. Eine wässerige, saure, empfängnisverhütende Zusammensetzung, dadurch gekennzeichnet, daß sie im wesentlichen aus einer wässerigen Lösung besteht, die folgende Stoffe enthält: 1,2 ml ±10% 2-hydroxy-propanol-Säure, 0,9 ml ±10% 3-hydroxy-3-carboxy-Pentanodial-Säure und 0,5 ml ±10% Eisessigsäure, V pro 100 ml, wobei die Lösung auf einen pH-Wert von 3,2 bis 3,5 eingestellt ist.

2. Ein Verfahren zur Herstellung einer wässerigen, sauren, empfängnisverhütenden Zusammensetzung nach Anspruch 1, dadurch gekennzeichnet, daß sie die Stufen der Lösung von 1,2 ml ±10%, 2-hydroxy-propanol-Säure, 0,9 ml ±10% 3-hydroxy-3-carboxy-pentanodiol-Säure und 0,5 ml ±10% Eisessigsäure in Wasser umfaßt, um die Lösung auf 100 ml zu bringen und dann den pH-Wert auf 3,2 bis 3,5 mit einer Natrium-hydroxid-Lösung einzustellen.

## Revendications

1. Une composition aqueuse, acidique, contraceptive caractérisée en ce qu'elle consiste essentiellement d'une solution aqueuse contenant 1,2 ml ±10% d'acide 2-hydroxy-propanoique, de 0,9 ml ±10% d'acide 3-hydroxy-3-carboxy-pentanodioique et de 0,5 ml ±10% d'acide acétique glacial par 100 ml; cette solution est ajustée a pH 3,2 à 3,5.

2. Une méthode pour la préparation d'une solution aqueuse, acidique, contraceptive selon la revendication 1 caractérisée en ce qu'elle comprend les stages de dissolution de 1,2 ml ±10% d'acide 2-hydroxy-propanoique, de 0,9 ml ±10% d'acide 3-hydroxy-3-carboxy-pentanodioique et 0,5 ml ±10% d'acide acétique glacial en eau, de porter la solution à 100 ml et d'ajuster la valeur pH à 3,2 à 3,5 par le moyen d'une solution de sodium hydroxide.

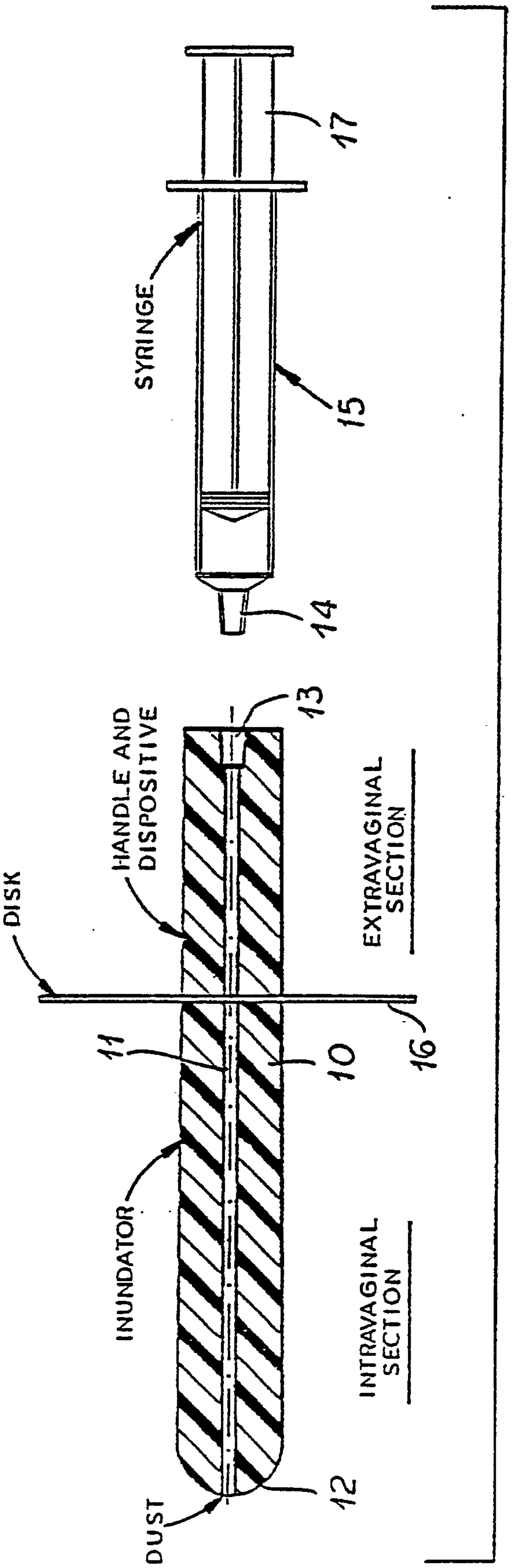
17
SYRINGE
15
14
13
HANDLE AND DISPOSITIVE
DISK
EXTRAVAGINAL SECTION
11
10
16
INUNDATOR
INTRAVAGINAL SECTION
DUST
12

FIG. 1

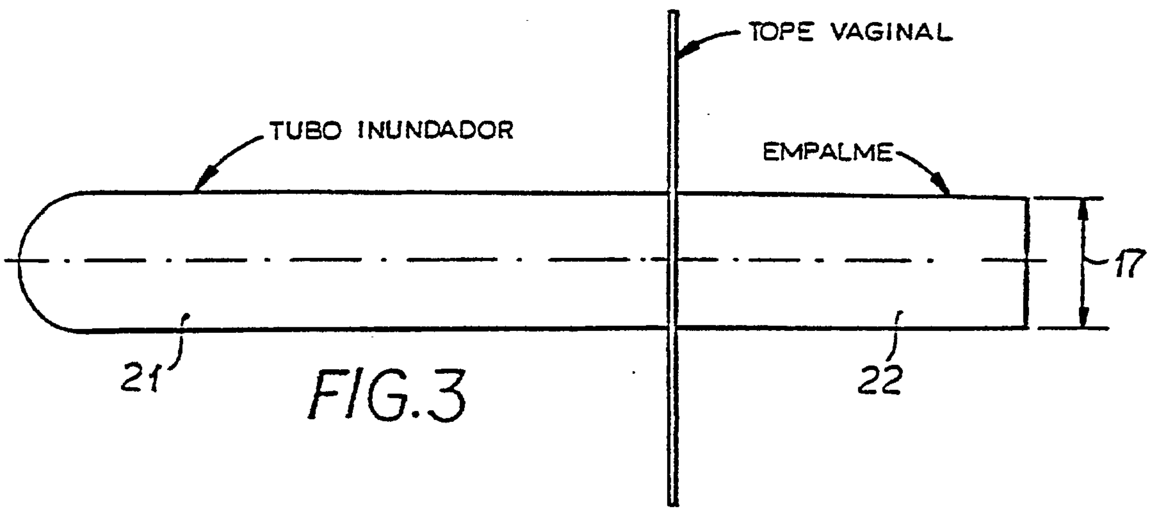


FIG.3

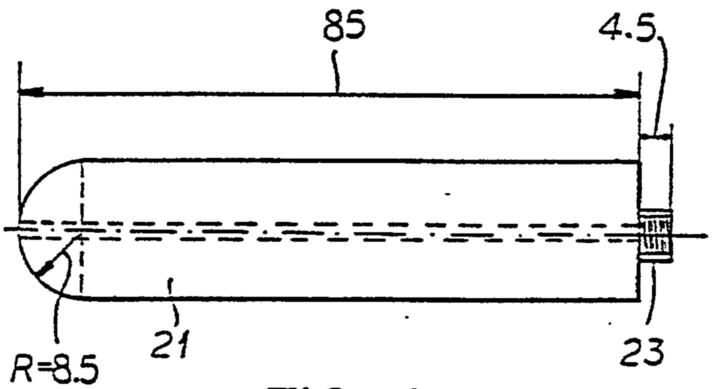


FIG.4

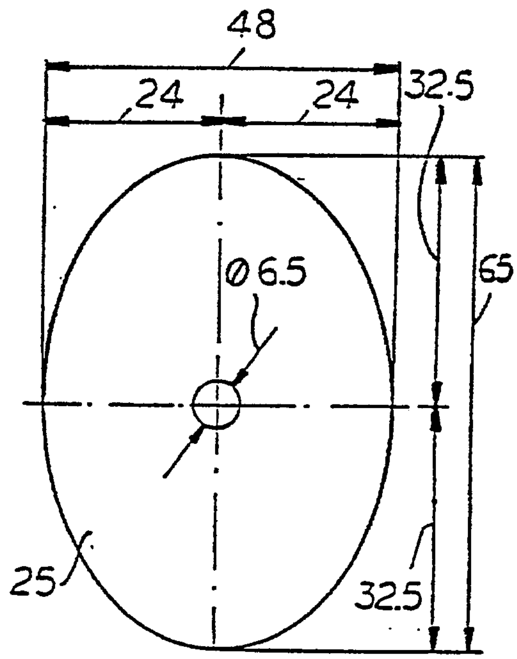


FIG.5

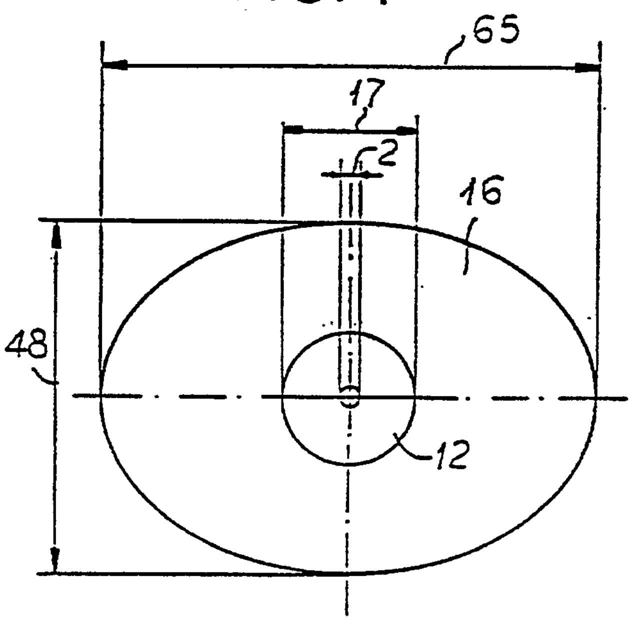


FIG.2

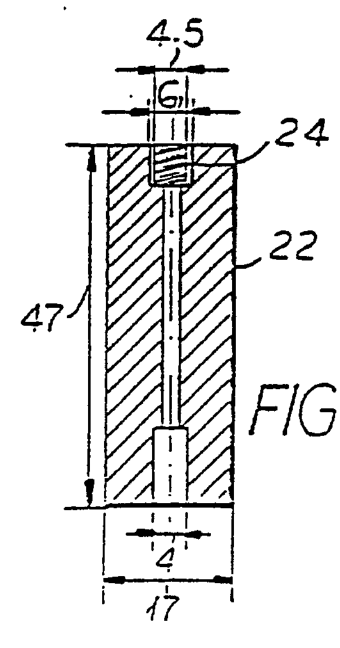


FIG.6